# EUROPEAN PATENT APPLICATION

(11) **EP 2 565 196 A1**
(43) Date of publication of application: **06.03.2013**
(21) Application number: 11774942.4
(22) Date of filing: 25.04.2011
(51) Int. Cl.: C07D 491/107, A61K 31/4188, A61P 27/16

(54) **PROPHYLACTIC OR THERAPEUTIC MEDICATION FOR INNER EAR DISORDERS**

(30) Priority: 28.04.2010 JP 2010102780
(71) Applicant: Sanwa Kagaku Kenkyusho Co., Ltd, Nagoya-shi, Aichi 461-8631 (JP); University of Tsukuba, Ibaraki 305-8577 (JP)
(72) Inventor: HARA, Akira, Tsukuba-shi Ibaraki 305-8577 (JP); TABUCHI, Keiji, Tsukuba-shi Ibaraki 305-8577 (JP); HIBI, Chihiro, Nagoya-shi Aichi 461-8631 (JP)
(74) Representative: Lenthall, Joseph
(86) International application number: PCT/JP2011/060025
(87) International publication number: WO 2011/136161

(57) **Abstract**

A pharmaceutical for preventing or treating an inner ear disorder, comprising a spirohydantoin derivative represented by a general formula (I): 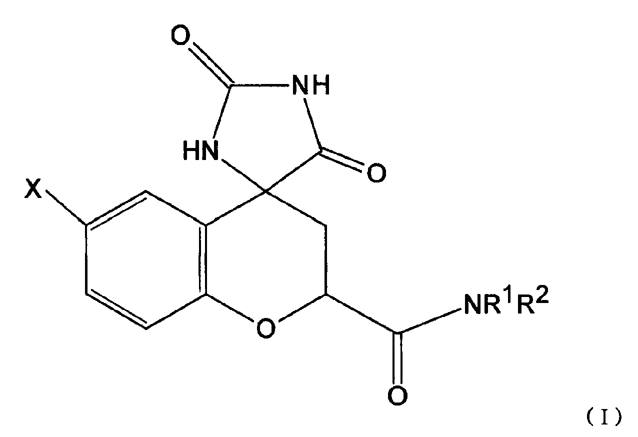 in the formula, X represents a halogen atom or a hydrogen atom, and R¹ and R² simultaneously or individually represent a hydrogen atom or an optionally substituted C₁₋₆ alkyl group,
or a pharmacologically acceptable salt thereof as an active ingredient.

## Description

### Technical Field

The present invention relates to a pharmaceutical for preventing or treating an inner ear disorder, comprising an aldose reductase inhibitor as an active ingredient.

### Background Art

Inner ear disorders mean that the inner ear has any disorder, and refer to a high frequency of disorders involving inner ear hearing loss, inner ear tinnitus, and vertigo due to inner ear disorder. Examples of the cause for inner ear hearing loss include aging, diseases, genetic factors, and noises. As of 2009, about 14% (38,000,000) of American adults are presumed to have a certain level of inner ear hearing loss. Inner ear tinnitus is a sensation of sound in the ear even when there is no sound source from outside. Vertigo due to inner ear disorder is severe rotary vertigo, and often involves nausea, vomiting, and tinnitus. So far, there has been no effective therapeutic pharmaceutical for inner ear hearing loss, inner ear tinnitus, and vertigo due to inner ear disorder.

Treatment of inner ear disorders is carried out by, for example, administration of a steroid, administration of a blood stream-improving agent (for example, adenosine triphosphate disodium) or a metabolism accelerator (for example, mecobalamin) for the purpose of improvement of inner ear circulatory disorders, administration of a diuretic (for example, isosorbide) for the purpose of improvement of endolymphatic hydrops, high pressure oxygen therapy, or stellate ganglion block therapy. However, the existing therapeutic means have limitations, and are not adequate for the treatment of inner ear disorders.

The spirohydantoin derivative represented by a general formula (I) is an aldose reductase (AR) inhibitor (Non-patent Document 1). The compound is used for diabetes complications (Patent Document 1), circulatory disorders (Patent Document 2), disorders due to aging as a Maillard reaction inhibitor (Patent Document 3), simple diabetic retinopathy (Patent Document 4), diabetic keratopathy (Patent Document 5), diabetic maculopathy (Patent Document 6), severe diabetic retinopathy (Patent Document 7), cardiac dysfunction or myocardial disorders (Patent Document 8), acute renal failure (Patent Document 9), cerebral ischemia or reperfusion injury following brain ischemia in cerebral apoplexy (Patent Document 10), and an agent for protecting retinal neurons or optic nerves (Patent Document 11). However, there is no report on the use for inner ear disorders. In addition, there is no report on the use of other aldose reductase inhibitors for inner ear disorders.

### Citation List

### Patent Literature

Patent Document 1: Japanese Patent Application Laid-Open Publication No. 61-200991
Patent Document 2: Japanese Patent Application Laid-Open Publication No. 4-173791
Patent Document 3: Japanese Patent Application Laid-Open Publication No. 6-135968
Patent Document 4: Japanese Patent Application Laid-Open Publication No. 7-242547
Patent Document 5: Japanese Patent Application Laid-Open Publication No. 8-231549
Patent Document 6: International Publication No. WO 2005/072066
Patent Document 7: International Publication No. WO 2005/079792
Patent Document 8: International Publication No. WO 2006/090699
Patent Document 9: International Publication No. WO 2007/069727
Patent Document 10: International Publication No. WO 2007/097301
Patent Document 11: International Publication No. WO 2008/093691

### Non-patent Literature

Non-patent Document 1: Arzneim.-Forsch./Drug Res., 44, 344-348 (1994)

### Summary of Invention

### Technical Problem

As mentioned above, for the prevention or treatment of inner ear disorders, the establishment of a therapy with high effectiveness and high safety is strongly demanded in the medical field. In particular, from the viewpoint of safety, a medicinal therapy with possible prolonged administration is strongly demanded. The present invention has been accomplished in view of these circumstances, and is intended to provide a pharmaceutical for preventing or treating inner ear disorders.

### Solution to Problem

Accordingly, the present inventors evaluated the effect of the spirohydantoin derivative represented by a general formula (I) for inner ear disorders. As a result of this, they found that the compound is effective on the decrease of function in auditory brainstem response (ABR), and loss of cochlear hair cells. More specifically, the present invention provides a pharmaceutical for preventing or treating inner ear disorder, comprising the spirohydantoin derivative represented by the general formula (I) as an active ingredient:

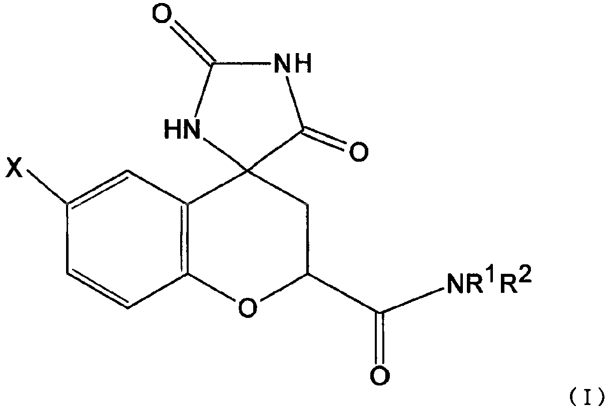

In the formula, X represents a halogen atom or a hydrogen atom, and R¹ and R² simultaneously or individually represent a hydrogen atom or an optionally substituted C₁₋₆ alkyl group which may be substituted.

The spirohydantoin derivative represented by a general formula (I) is preferably (2S,4S)-6-fluoro-2',5'-dioxospiro[chroman-4,4'-imidazolidine]-2-carboxa mide (hereinafter referred to as fidarestat).

Specific examples of inner ear disorder include inner ear hearing loss, inner ear tinnitus, and vertigo due to inner ear disorder. Examples of inner ear hearing loss include sensorineural and mixed hearing loss.

The compound of the present invention may be administered in combination with other pharmaceutical for inner ear disorders. Examples of the other pharmaceutical for inner ear disorders include a steroid, an anticholinergic agent, an antihistamine agent, an antiviral agent, a leukotriene receptor antagonist, an anticoagulation agent, a vasodilator agent, a clot busting agent, a vitamin B, a vitamin B derivative, and an inner ear circulation-improving agent. The compound of the present invention may be combined with one or more of the above pharmaceuticals.

### Advantageous Effect of Invention

The present invention provides a pharmaceutical for preventing or treating inner ear disorders. According to a preferred embodiment of the present invention, a medicinal therapy with possible prolonged administration and high safety is provided.

### Brief Description of Drawings

FIG. 1 shows the effect on increasing the auditory brainstem response (ABR) threshold at one week after the preventive administration of fidarestat in high-level noise exposure mice model.
FIG. 2 shows the effect on the auditory brainstem response (ABR) threshold elevation at two weeks after the preventive administration of fidarestat in high-level noise exposure mice model.
FIG. 3 shows the effect on the auditory brainstem response (ABR) threshold elevation at one week after the therapeutic administration of fidarestat, prednisolone, or the combination of them in high-level noise exposure mice model.
FIG. 4 shows the effect on the auditory brainstem response (ABR) threshold elevation at two weeks after the therapeutic administration of fidarestat, prednisolone, or the combination of them in high-level noise exposure mice model.
FIG. 5 shows the inhibitory effect of fidarestat on increase of the loss rate of cochlear outer hair cells.
FIG. 6 shows the inhibitory effect of fidarestat on gentamicin-induced loss of outer hair cells.

### Description of Embodiments

Hereinafter the present invention will be explained in more detail. An active ingredient of the pharmaceutical according to the present invention is a spirohydantoin derivative represented by the general formula (I), or a pharmacologically acceptable salt thereof. Both of them are known compounds, and can be prepared according to the synthetic method described in, for example, Japanese Patent Application Laid-Open Publication No. 63-057588.

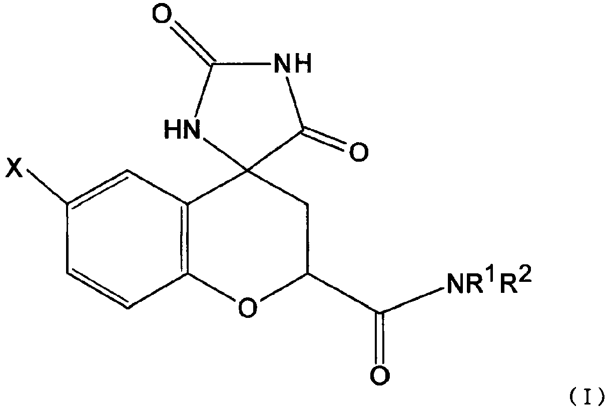

X in the formula represents a halogen atom or a hydrogen atom, preferably a halogen atom such as a fluorine atom. R¹ and R² in the formula simultaneously or individually represent a hydrogen atom or an optionally substituted C₁₋₆ alkyl group, preferably a hydrogen atom. Of the C₁₋₆ alkyl groups, a C₁₋₃ alkyl group is preferred. Among these compounds, a compound wherein X is a fluorine atom and R¹ and R² are hydrogen atoms is particularly preferred.

The pharmacologically acceptable salt of the spirohydantoin derivative is preferably a pharmaceutically acceptable salt in consideration of its use. Specific examples include pharmacologically acceptable inorganic and organic base salts such as sodium, lithium, potassium, magnesium, ammonium, quaternary ammonium salts, diethylamine, and diethanolamine. These salts are readily obtained by, for example, treating the spirohydantoin derivative represented by the general formula (I) with a base.

The spirohydantoin derivative has four stereoisomers. The present invention includes these isomers and racemic bodies which are mixtures of them. Among them, (2S,4S)-6-fluoro-2',5'-dioxospiro[chroman-4,4'-imidazolidine]-2-carboxa mide (general name: fidarestat) is preferred.

The pharmaceutical of the present invention is the pharmaceutical for preventing or treating inner ear disorders. Specific examples of inner ear disorders include inner ear hearing loss, inner ear tinnitus, and vertigo due to inner ear disorder. Inner ear disorders mean that the inner ear is damaged by any factor. The inner ear is the deepest part of the whole ear. The mammalian inner ear is composed of cochlea, vestibule, and semicircular canal. Examples of the cause for inner ear disorders include circulatory disorders, metabolism disorders, and inflammation (infection diseases). Examples of tissue signs include loss or degeneration of hair cells, and endolymph caused by abnormal lymph circulation within the inner ear. The inner ear contains organs responsible for senses of hearing and equilibrium. Therefore, when the inner ear has any disorder, symptoms and signs such as hearing loss, tinnitus, vertigo, nystagmus, stagger, ear pain, feeling of fullness in the ear, autophony, or hyperacusia, occur.

Examples of the cause for hearing loss include aging, diseases, genetic factors, and noises. The hearing loss is classified into conductive, sensorineural and mixed (mixed conductive and sensorineural) hearing loss, according to the cause of the disorder. Conductive hearing loss is due to external or middle ear disorder, and occurs when an organ transmitting vibrations is inflamed. Sensorineural hearing loss is the disorder of the inner ear cochlea which receives sound vibrations, and is caused by depressed sensory cell function or neural disorder during signal transfer from sensory cells to the brain. Sensorineural hearing loss also occurs when hair cells within the cochlea are decreased by nerve destruction or aging caused by a certain cause. Thus, inner ear hearing loss normally stands for sensorineural hearing loss or mixed hearing loss.

Examples of the sensorineural hearing loss include sudden deafness, the cause of which is still unknown, viral infection, drug-induced deafness caused by side effects of an antibiotic, anticancer agent, or diuretic, noise deafness caused by exposure to a loud noise for a long period of time, acoustic trauma deafness caused by an explosion sound, gunshot sound, or a high volume headphone, deafness caused by Meniere's disease, and senile deafness because of aging.

In the present invention, inner ear hearing loss means difficulty in hearing sound or voice due to the decreased sense of hearing caused by inner ear damage inflicted by any factor, Inner ear hearing loss includes sensorineural hearing loss and mixed hearing loss (mixed conductive and sensorineural hearing loss). Inner ear tinnitus means a sensation of sound in the ear even when there is no sound source from outside. Inner ear hearing loss is often accompanied by tinnitus, and the protection of the inner ear from inner ear hearing loss contributes to the prevention of tinnitus.

The pharmaceutical according to the present invention has a protective effect on hair cells loss in the inner ear, and thus likely relieves vertigo due to inner ear disorder. Vertigo due to inner ear disorder is often severe rotary vertigo, and often involves nausea, vomiting, and tinnitus. Benign paroxysmal positional vertigo and Meniere's disease are also included in vertigo due to inner ear disorder. The organ responsible for sense of hearing (cochlea) and the organ responsible for equilibrium related with vertigo (vestibulum in inner ear) are readily influenced by each other, because they are developed and differentiated from the same organ, and are located at the same position or very close to each other. Therefore, inner ear disorders often involve hearing loss as well as tinnitus and vertigo. Accordingly, improvement of inner ear disorders is likely effective for hearing loss, tinnitus, and vertigo.

The dose of the compound represented by the general formula (I), which is an active ingredient of the pharmaceutical according to the present invention, depends on, for example, the symptom, age, administration method, and dosage form. In clinical cases, the dose for one adult patient is normally from 0.1 mg to 450 mg, preferably from 1 mg to 300 mg a day, which is taken all at once or divided into several doses for consecutive days.

The pharmaceutical according to the present invention is formulated into, for example, a tablet, a capsule agent, a powder, a granular agent, a liquid agent, a syrup agent, an injection, or an eardrop by any ordinary preparation technique. The formulated pharmaceutical may be administered orally or parenterally.

When the pharmaceutical is in a solid agent, it may include a pharmacologically acceptable excipient such as starch, lactose, refined white sugar, glucose, crystalline cellulose, carboxy cellulose, carboxymethyl cellulose, carboxy ethyl cellulose, calcium phosphate, magnesium stearate, or gum arabic. As necessary, the medication may contain, for example, a lubricant, a binder, a disintegrating agent, a coating agent, and a coloring agent. When the pharmaceutical is in a liquid agent, it may include a stabilizer, a solubilizing agent, a suspending agent, an emulsifying agent, a buffer, and a preservative.

Furthermore, the pharmaceutical according to the present invention may be administered in combination with other pharmaceutical or therapy used for inner ear disorders. Examples of the other pharmaceutical which may be combined include the steroid, the anticholinergic agent, the antihistamine agent, the antiviral agent, the leukotriene receptor antagonist, the anticoagulation agent, the vasodilator agent, the clot busting agent, the vitamin B, the vitamin B derivative, and the inner ear circulation-improving agent. The pharmaceutical according to the present invention may be combined with one or more of other pharmaceuticals. The other medications for inner ear disorder are administered mainly for improving blood circulation in the inner ear. The combination of the pharmaceutical according to the present invention with the other pharmaceutical for inner ear disorders can enhance the effects of the respective pharmaceuticals. In particular, the dose and administration period of steroids are limited because of clinical side effects. The combination with the pharmaceutical according to the present invention brings great advantages from the viewpoint of safety and efficacy, such as early withdrawal of steroids.

Examples of the other therapy which may be combined include high-pressure oxygen therapy and stellate ganglion block therapy. High-pressure oxygen therapy is carried out by inhaling oxygen under a pressure higher than atmospheric pressure, thereby delivering oxygen throughout the body, and is intended to improve blood stream. Stellate ganglion block therapy is carried out by blocking stellate ganglion to temporarily paralyze the sympathetic nerve function, thereby improving the activity of parasympathetic nerve.

When the pharmaceutical according to the present invention is administered in combination with other pharmaceutical for inner ear disorder, the active ingredients may be independently contained in plural preparations, or in one and the same preparation. The combined pharmaceutical is composed of plural pharmaceuticals or active ingredients. The plural pharmaceuticals or active ingredients are preferably administered to a patient substantially at the same time. When the plural active ingredients to be administered are contained in different preparations, these plural preparations may be not always administered at the same time. In normal cases, these preparations are administered according to their original administration method. Therefore, the number of doses may be identical or different.

Examples of the administration of the combination of the pharmaceutical according to the present invention and other pharmaceutical for inner ear disorders include the followings:
(1) administration of a single preparation containing both the pharmaceutical according to the present invention and other pharmaceutical;
(2) simultaneous administration of two or more preparations through the same administration route, the preparations being obtained by individually formulating the pharmaceutical according to the present invention and other pharmaceutical;
(3) administration of two or more preparations with a time lag through the same administration route, the preparations being obtained by individually formulating the pharmaceutical according to the present invention and other pharmaceutical;
(4) simultaneous administration of two or more preparations through different administration routes, the preparations being obtained by individually formulating the pharmaceutical according to the present invention and other pharmaceutical;
(5) administration of two or more preparations with a time lag through different administration routes, the preparations being obtained by individually formulating the pharmaceutical according to the present invention and other pharmaceutical;
(6) administration of two or more preparations at different intervals through the same administration route, the preparations being obtained by individually formulating the pharmaceutical according to the present invention and other pharmaceutical; and
(7) administration of two or more preparations at different intervals through different administration routes, the preparations being obtained by individually formulating the pharmaceutical according to the present invention and other pharmaceutical.

The dose of other pharmaceutical for inner ear disorders depends on the drug. The dose of the steroid is normally from 0.1 mg to 500 mg a day, which is taken all at once or divided into several doses. The anticholinergic agent, the antihistamine agent, the antiviral agent, the leukotriene receptor antagonist, the anticoagulation agent, the vasodilator agent, the clot busting agent, the vitamin B, the vitamin B derivative, and the inner ear circulation-improving agent are preferably administered according to the normal administration methods and doses used for the respective medications.

The spirohydantoin derivative represented by the general formula (I), which is an active ingredient of the pharmaceutical according to the present invention, is a compound having an aldose reductase (AR) inhibitory activity. It is thus suggested that the AR inhibitory activity is related with prophylactic or therapeutic effect for inner ear disorders. Examples of other compound having an aldose reductase inhibitory activity include ranirestat (AS-3201), ARI-809 (CP-744809), epalrestat, zopolrestat, zenarestat, tolrestat, imirestat, ponalrestat, voglistat, [5-(3-thienyl)tetrazole-1-yl]acetic acid monohydrate (TAT), M-160209, SG-210, and NZ-314. These compounds also likely have a prophylactic or therapeutic effect for inner ear disorders.

### Examples

The present invention will be explained in more detail by the examples. In the following examples, fidarestat was selected from the compounds represented by the general formula (I), and tested. These examples will not limit the present invention, and may be modified without departing from the spirit according to the present invention.

### 1. Evaluation of inner ear disorders in high-level noise exposure mice models

### Test Method

The test was carried out using high-level noise exposure mice. These mice are inner ear disorder models exposed to high-level noise, and prepared by exposing B6 mice (Slc, female, test was started on 8 weeks old) to high-level noise (128 dB SPL (sound pressure level)) for 4 hours. The auditory brainstem response (ABR) threshold was measured immediately before the exposure to high-level noise (initial value). At 1 week and 2 weeks after the exposure for 4 hours, the ABR was measured. For the prevention test, after measuring the ABR threshold at 2 weeks after, the inner ears were excised and fixed so as to allow pathological assessment of the loss of two types of hair cells, or inner and outer hair cells within the cochlea. The pathological assessment of the hair cell loss was carried out on the inner hair cells (one line of inner hair cells is represented by IHC) and outer hair cells (three lines of outer hair cells are represented by OHC 1, 2, and 3 from the inside to outside). The verification was carried out by analysis of variance (Fisher's test as post-hoc test).

### (1) Prophylactic administration of pharmaceutical

The mice were divided into two groups: a group exposed to high-level noise (control group, n = 10), and a group exposed to high-level noise and administrated with fidarestat at a dose of 32 mg/kg/day (fidarestat-administrated group, n = 10). The ABR and pathological assessment were carried out in 10 and 5 mice of each group, respectively. Fidarestat was administered mixed with the feed during the period from two days before to two weeks after the exposure to high-level noise.

### (2) Therapeutic administration of pharmaceutical

The mice were divided into five groups: a group exposed to high-level noise (control group, n = 6), two group exposed to high-level noise and administrated with fidarestat at doses of 8 mg/kg/day or 32 mg/kg/day (fidarestat-administrated groups, n = 6), a group exposed to high-level noise, and administrated with prednisolone at a dose of 1 mg/kg/day (prednisolone-administrated group, n = 6), and a group exposed to high-level noise, and administrated of combination with prednisolone at a dose of 1 mg /kg/day and fidarestat at a dose of 32 mg/kg/day (combination use group, n = 6). The ABR was carried out in 6 mice of each group. Fidarestat was administered mixed with the feed during the period immediately after to two weeks after the exposure to high-level noise. Prednisolone was orally administered immediately after the exposure to high-level noise.

### Result

### (1) Effect on increasing the auditory brainstem response (ABR) threshold

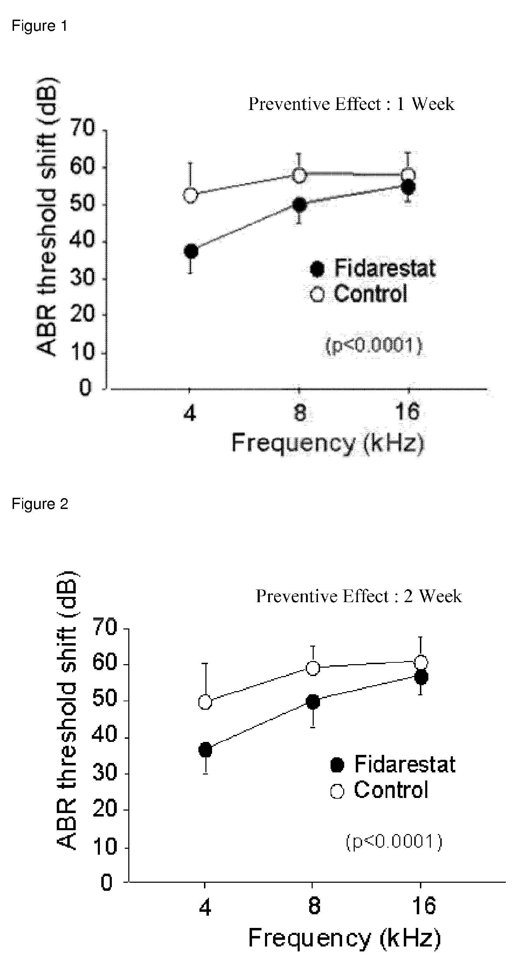

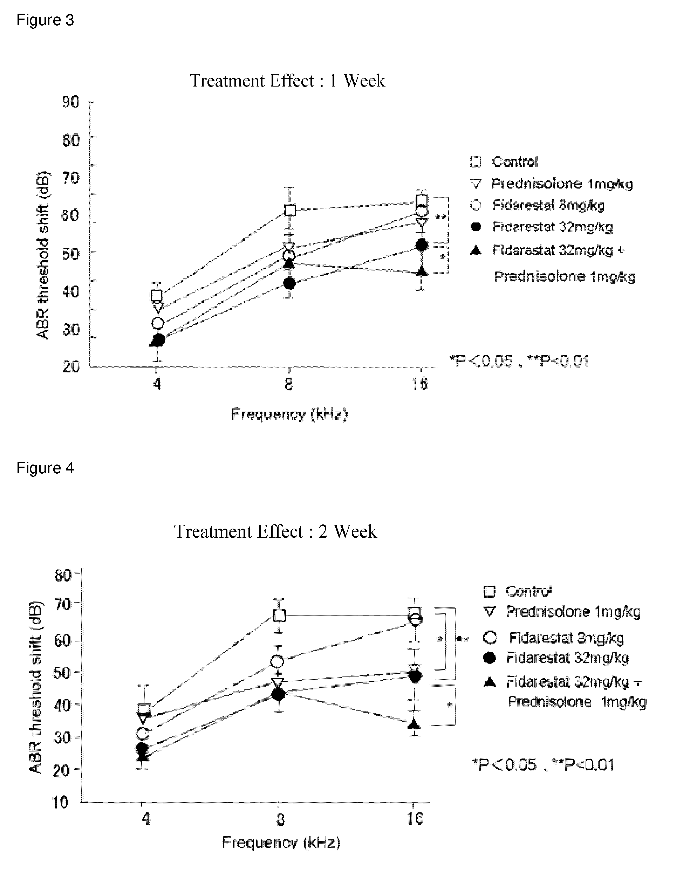

Fidarestat significantly inhibited to increase the auditory brainstem response (ABR) threshold, which is caused by exposure to high-level noise, at both the points one week after and two weeks after the exposure to high-level noise (FIGs. 1-4). More specifically, fidarestat exhibited prophylactic and therapeutic effects on the ABR threshold increase caused by the exposure to high-level noise. Regarding the therapeutic effect, the fidarestat-administrated group at a dose of 32 mg /kg/day observed an equivalent effect to the prednisolone-administrated group at a dose of 1 mg /kg/day, and a more significant inhibitory effect was observed in the combination use group as compared to the prednisolone-administrated group.

### (2) Effect on increasing the loss rate of cochlear hair cells

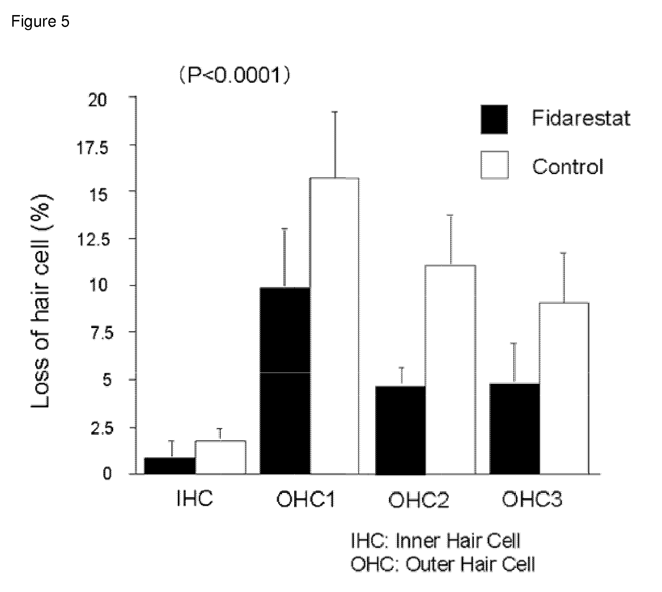

Fidarestat significantly inhibited to increase the loss rate of outer hair cells observed at two weeks after the exposure to high-level noise (FIG. 5).

### Discussion

The effect of fidarestat on inner ear disorders was studied using high-level noise exposure in mice model, which are widely used model for the assessment of efficacy on inner ear disorders. Acoustic-induced trauma models are inflammatory models caused by free radicals, active oxygen, and phospholipase A2, and are known to be effectively treated by steroids. Fidarestat showed a significant protective effect on increasing the auditory brainstem response (ABR) threshold and increasing the loss rate of cochlear outer hair cells, which are observed after exposure to high-level noise. The pathological effect within the cochlea agreed with the effect on the auditory function. The fact that fidarestat showed a protective effect on the inner ear suggests that fidarestat is effective on inner ear disorders such as inner ear hearing loss, vertigo due to inner ear disorder, and inner ear tinnitus. Furthermore, the effect was equivalent to that of the result of prednisolone, which is a steroid widely used for inner ear disorders. It was also showed that the combination of fidarestat and prednisolone achieved a higher effect as compared to the single administration of either of the compound alone. Withdrawal of steroids such as prednisolone is studied from the viewpoint of clinical side effects. The present test results suggest that fidarestat is effective as a maintenance therapeutic agent after withdrawal of steroids. Accordingly, fidarestat is effective for the prevention or treatment of inner ear disorders.

### 2. Evaluation of inner ear disorders in gentamicin-induced loss of outer hair cells model

### Test method

After excision of the cochlea from SD rats of 3-5 days old, lateral walls were removed, and then the basal turn of the organ of Corti was extracted. The organ of Corti was incubated overnight, and then cultured in culture solutions containing gentamicin (35 µg) and fidarestat at different concentrations (0, 0.05, 0.1, 0.2, 0.5, 1, and 2 µM) for 48 hours. After culturing, the organ of corti was fixed with 4% paraformaldehyde. The culture solution was a Dulbecco's modified eagles medium (DMEM solution) containing 10% fetal bovine serum, HEPES (250 mM), and penicillin G (30 U/l), and the organ was cultured at 37°C, 5%CO_{2,} at a humidity of 95%.

### Test result

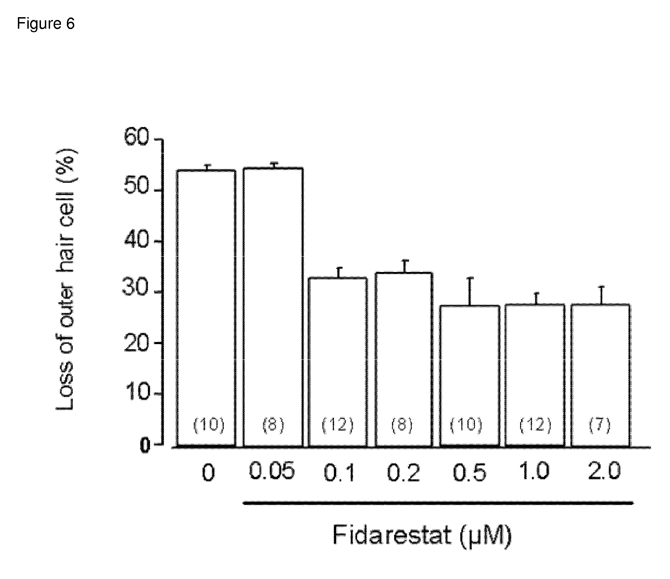

The loss rate of gentamicin-induced outer hair cells loss was 50% in the control group, but significantly reduced in the fidarestat-administrated group at the concentration of 0.1 µM, and the loss rate at the concentrations of 0.5 µM or more was 25% (FIG. 6). In other words, 75% outer hair cells were survival at the concentrations of 0.5 µM or more.

### Discussion

The effect of fidarestat on drug-induced inner ear disorders was studied using gentamicin-induced loss of hair cells models, which are widely used for the assessment of efficacy on drug-induced inner ear disorders. As a result of this, fidarestat inhibited the gentamicin-induced loss of hair cells at a low concentration, indicating its efficacy. In other words, fidarestat is effective for the prevention or treatment of drug-induced inner ear disorders caused by side effects of antibiotics, anticancer agents, or diuretics. As described above, fidarestat was effective both on high-level noise-induced inner ear disorders and on drug-induced inner ear disorders, indicating that fidarestat is effective on inner ear disorders irrespective of the cause.

## Claims

1. A pharmaceutical for preventing or treating an inner ear disorder, comprising a spirohydantoin derivative represented by a general formula (I): 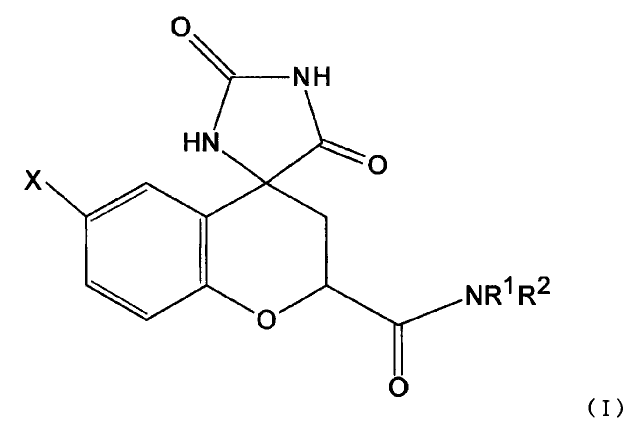 in the formula, X represents a halogen atom or a hydrogen atom, and R¹ and R² simultaneously or individually represent a hydrogen atom or an optionally substituted C₁₋₆ alkyl group,
or a pharmacologically acceptable salt thereof as an active ingredient.

2. The pharmaceutical according to claim 1, wherein the spirohydantoin derivative represented by the general formula (I) is (2S,4S)-6-fluoro-2',5'-dioxospiro[chroman-4,4'-imidazolidine]-2-carboxa mide.

3. The pharmaceutical according to claim 1 or 2, wherein the inner ear disorder is selected from the group consisting of inner ear hearing loss, inner ear tinnitus, and vertigo due to inner ear disorder.

4. The pharmaceutical according to claim 3, wherein the inner ear hearing loss is sensorineural hearing loss or mixed hearing loss.

5. The pharmaceutical according to any one of claims 1 to 4, which composed of a combination with other pharmaceutical for inner ear disorder.

6. The pharmaceutical according to claim 5, wherein the other pharmaceutical for inner ear disorder is at least one kind selected from the group consisting of a steroid, an anticholinergic agent, an antihistamine agent, an antiviral agent, a leukotriene receptor antagonist, an anticoagulation agent, a vasodilator agent, a clot busting agent, a vitamin B, a vitamin B derivative, and an inner ear circulation-improving agent.
